Europäisches Patentamt

European Patent Office

Office européen des brevets

(11) Veröffentlichungsnummer: **0 261 331 B1**

# (12) EUROPÄISCHE PATENTSCHRIFT

(45) Veröffentlichungstag der Patentschrift: **18.09.91**

(51) Int. Cl.⁵: **C07C 309/20, C25D 3/16**

(21) Anmeldenummer: **87110046.7**

(22) Anmeldetag: **11.07.87**

Die Akte enthält technische Angaben, die nach
dem Eingang der Anmeldung eingereicht wurden
und die nicht in dieser Patentschrift enthalten sind.

(54) **Alpha-Hydroxi Propinsulfonsäure und deren Salze, saure Nickelbäder enthaltend diese Verbindungen sowie Verfahren zu ihrer Herstellung.**

(30) Priorität: **22.09.86 DE 3632514**

(43) Veröffentlichungstag der Anmeldung:
**30.03.88 Patentblatt 88/13**

(45) Bekanntmachung des Hinweises auf die
Patenterteilung:
**18.09.91 Patentblatt 91/38**

(84) Benannte Vertragsstaaten:
**BE CH DE ES FR GB IT LI LU NL SE**

(56) Entgegenhaltungen:
**AT-E- 6 873          DE-A- 2 544 729**
**DE-B- 2 054 221      DE-B- 2 215 737**
**US-A- 4 062 738      US-A- 4 435 254**

**CHEMICAL ABSTRACTS, Band 90, Nr. 22,28.**
**Mai 1979, Columbus, Ohio, US; ZEIMYTE, O.**
**et al.: "Effect of sulfates of unsaturated com-**
**pounds on the electroplating of nickel", Sei-**
**te 571**

(73) Patentinhaber: **SCHERING AKTIENGESELL-**
**SCHAFT Berlin und Bergkamen**
**Müllerstrasse 170/178 Postfach 65 03 11**
**W-1000 Berlin 65(DE)**

(72) Erfinder: **Dahms, Wolfgang**
**Hermsdorfer Strasse 53A, No. 1**
**Berlin 26(DE)**
Erfinder: **Schulz, Klaus-Dieter**
**Feldstrasse 54**
**W-1000 Berlin 20(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft α-Hydroxipropinsulfonsäure und deren Salze, saure Nickelbäder enthaltend diese Verbindungen sowie Verfahren zu ihrer Herstellung.

Es ist seit langem bekannt, daß sauren, insbesondere schwach sauren Nickelelektrolyten bestimmte organische Substanzen in geringen Mengen zugesetzt werden, um statt einer matten Abscheidung eine glänzende Nickelabscheidung zu erhalten.

Eine Gruppe dieser Zusätze, auch Grundglänzer genannt, sind ungesättigte, meist aromatische Sulfonsäuren, Sulfinsäuren, Sulfonamide oder Sulfimide beziehungsweise deren Salze ,und die bekanntesten Verbindungen sind zum Beispiel m-Benzoldisulfonsäure und Benzoesäuresulfimid.

Diese Verbindungen erzeugen, allein dem galvanischen Bad zugesetzt, über einen gewissen Stromdichtebereich einen glänzenden, aber nicht eingeebneten Niederschlag. Ihre alleinige Anwendung hat deshalb keinerlei praktische Bedeutung, da die Qualität der mit ihnen erhaltenen Nickelüberzüge nicht den heutigen Anforderungen entspricht.

Ihr offensichtlicher Mangel ist die Fähigkeit, rauhe Oberflächen einzuebnen, ohne den Niederschlag zu verspröden.

Es sind auch schon Verbindungen bekannt geworden, die eingeebnete Niederschläge erzeugen werden. Sie werden in der Regel zusammen mit einem oder mehreren Grundglänzern angewendet.

Bekannte Einebner sind zum Beispiel dreifach ungesättigte Alkohole oder dreifach ungesättigte Amine. Sie zeigen vor allem beim Dauerbetrieb und schon bei leichter Überdosierung dunkle Abscheidungen im niedrigen Stromdichtebereich. Häufig tritt sogar der Fall ein, daß keine Abscheidung im niedrigen Stromdichtebereich auftritt und des Untergrundmaterial sichtbar bleibt.

Des weiteren sind als Einebner quarternäre, aromatische stickstoffhaltige Verbindungen wie Pyridinium- oder Chinoliniumverbindungen als Einebner vorgeschlagen worden, bei denen jedoch die einebnende Wirkung nur auf den hohen Stromdichtebereich beschränkt ist.

Allen Einebnern ist gemeinsam, daß sie entweder schon beim Ansatz, meist aber im Dauerbetrieb den Nickelniederschlag verspröden.

Zu den mit einebnender Wirkung bestimmten Stoffen gehören außerdem wie zum Beispiel Propargylaldehyd, der jedoch den Nachteil hat, zu völlig ungedeckten Stellen im Bereich der niedrigen Stromdichte zu führen und im mittleren Stromdichtebereich eine Stufenabscheidung (Relief) zu erzeugen. Außerdem ist er chemisch unstabil und kann nicht in wässriger Lösung gelagert werden.

Die vorliegende Erfindung hat die Aufgabe, Verbindungen für galvanische Bäder, insbesondere saure Nickelbäder, zu schaffen, die sowohl eine gute Einebnung als auch hervorragende Glanzbildung insbesondere bei niedrigen Stromdichten ermöglichen.

Diese Aufgabe wird durch die in den Ansprüchen 1 bis 4 gekennzeichneten Verbindungen gelöst.

Die erfindungsgemäßen Verbindungen bewirken in sauren Nickelbädern überraschenderweise sowohl eine gute Tiefenstreuung als auch eine herausragende Einebnung und vermeiden darüberhinaus auch bei längerem Betrieb der Bäder die Bildung spröder Niederschläge.

Die erfindungsgemäß neuen Verbindungen sind im Bad als solche enthalten, wobei es unerheblich ist, ob sie bereits bei der Herstellung des Bades diesem als solche hinzugefügt oder aufgrund von chemischen Reaktionen aus anderen Verbindungen im Bad gebildet werden:
Die Herstellung der erfindungsgemäßen Verbindungen erfolgt zum Beispiel dadurch, daß man Propargylaldehyd in wässriger Lösung mit Alkali- oder Ammoniumhydrogensulfit in äquivalenten Mengen bei Raumtemperatur zur Reaktion bringt und die Reaktionsprodukte in an sich bekannter Weise isoliert.

α-Hydroxipropinsulfonsäure kann als solche oder in Form ihrer Salze, zum Beispiel der Alkalisalze, vorzugsweise Natrium-oder Kaliumsalz oder des Ammoniumsalzes verwendet werden.

Das Bad wird zur Abscheidung von Nickelniederschlägen unter Zusatz der erfindungsgemäßen Verbindungen im allgemeinen eine Nickelsalzlösung benutzt, der man zusätzlich eine schwache Säure zur Pufferung zusetzt.

In der Praxis wird zweckmäßigerweise ein Watt sches Bad verwendet, das folgende Zusammensetzung hat:
200 - 400 g/l Nickelsulfat ($NiSO_4 \cdot 7H_2O$)
30 - 200 g/l Nickelchlorid ($NiCl_2 \cdot 6H_2O$)
30 - 50 g/l Borsäure ($H_3BO_3$)
Das Bad kann einen pH-Wert von 3 bis 5,5 , vorzugsweise von 4 bis 5, aufweisen. Die Temperatur kann zur Erhöhung der Stromdichten bis zu 75°C betragen, in der Regel liegt sie zwischen 50° und 60°C.

Hochleistungsbäder haben einen Chloridgehalt von 10 - 50 g/Liter und zeigen beim Verwenden der erfindungsgemäßen Verbindungen die besten Ergebnisse. Das Nickelchlorid kann teilweise oder ganz durch Natriumchlorid ersetzt werden. Die Stromdichte kann bei 55°C bis zu 10 A/dm$^2$ betragen.

Die erfindungsgemäßen Verbindungen wirken überraschenderweise nicht wie bekannte Einebner bevorzugt im hohen Stromdichtebereich, sondern auch im mittleren bis niedrigen Stromdichtebereich einebnend. Der weitere wesentliche Vorteil ist, daß selbst bei starkem Dosieren der erfindungsgemäßen Verbindungen die Niederschläge im Gegensatz zu den bekannten Einebnern keine dunkle Nickelabscheidung, die meist in der niedrigen Stromdichte auftritt, erzeugen.

Die erfindungsgemäßen Verbindungen entfalten zwar schon für sich allein hervorragende Wirkungen, optimale Ergebnisse erzielt man jedoch insbesondere durch Kombination mit bekannten Grundglänzern und Einebnern. Man erzielt auf dieser Weise so über den gesamten für die Praxis erforderlichen Stromdichtebereich eine ausgezeichnete Einebnung, ohne daß die Niederschläge die bekannten Nachteile der dunklen Abscheidung zeigen.

Die Konzentration der erfindungsgemäßen Verbindungen im Nickelbad ist sehr gering und kann von 0,005 bis 5 g/Liter vorzugsweise von 0,05 und 0,2 g /Liter betragen. Niedrige Konzentrationen sind dann vorteilhafterweise anzuwenden, wenn die erfindungsgemäßen Verbindungen in Kombination von Grundglänzern und Einebne rn auf Basis von Pyridiniumverbindungen angewendet werden.

Die Grundglänzer werden in der Regel in Mengen von 0,1 - 10 g/Liter dem Elektrolyten zugesetzt. Einebner, insbesondere Pyridiniumverbindungen, werden zweckmäßigerweise zwischen 0,02 und 0,25 g/Liter dosiert. Netzmittel zur Verhinderung einer porigen Abscheidung können in Mengen bis zu 10 g/Liter zugesetzt werden.

Die folgenden Beispiele dienen zur Erläuterung der Erfindung.

BEISPIEL 1

HERSTELLUNG VON α-HYDROXIPROPINSULFONSÄURE, NATRIUMSALZ

26 g einer 40%iger Natriumhydrogensulfit-Lösung ($\triangleq$ 0,1 mol $NaHSO_3$) werden in einem 250 ml Rührkolben vorgelegt. Dazu tropft man 5,4 g Propargylaldehyd ($\triangleq$ 0,1 mol $HC\equiv C\text{-}CHO$) innerhalb von 10 Minuten. Die Umsetzung geschieht bei Raumtemperatur und ist nach weiteren 20 Minuten abgeschlossen.

Zur Isolierung wird die Reaktionslösung bei 50° C und 20 Torr am Rotationsverdampfer eingeengt. Die Ausbeute beträgt 97% der Theorie.

α-Hydroxipropinsulfonsäure, Natriumsalz, Fp: 130° C (Zersetzung)

Die Charakterisierung erfolgte durch NMR und IR Spektren bezogen auf den Standard 3-(Trimethylsilyl)-propionsäure, Natriumsalz (TMS) in $D_2O$ als Lösungsmittel.

$\delta$ = 3,05 ppm ($TMS/D_2O$)

$\delta$ = 5,1 ppm (($TMS/D_2O$)

BEISPIEL 2

HERSTELLUNG VON α-HYDROXIPROPINSULFONSÄURE, KALIUMSALZ

Bei der Herstellung des Kaliumsalzes werden 28 g einer 40 %igen Kaliumbisulfit-Lösung ($\triangleq$ 0,05 mol $K_2S_2O_5$) vorgelegt. Nach der Reaktion wird das Kaliumsalz bei 40° C und 20 Torr eingeengt. Die Ausbeute beträgt 95 %.

α-Hydroxipropinsulfonsäure, Kaliumsalz, Fp: 134° C (Zersetzung)

In der Hull-Zelle zeigt diese Substanz die gleiche Wirkung wie das Natriumsalz.

Die Spektren sind analog denen des Natriumsalzes.

BEISPIEL 3

HERSTELLUNG VON α-HYDROXIPROPINSULFONSÄURE, AMMONIUMSALZ

Bei der Herstellung des Ammoniumsalzes wird zunächst Ammoniumdisulfit durch Einleiten von Schwefeldioxyd in Ammoniaklösung hergestellt (siehe GMELIN, NH - Band, 23, Seiten 259-261).

Hierbei wird 23 g einer 40%igen Ammoniumdisulfit-Lösung ($\triangleq$0,05 mol $(NH_4)_2S_2O_3$) eingesetzt. Die Ausbeute beträgt 80 %.

In der Hull-Zelle zeigt diese Substanz die gleiche Wirkung wie das Natriumsalz.

Die Spektren sind analog denen des Natriumsalzes.

α-Hydroxipropinsulfonsäure, Ammoniumsalz, Fp: ab 121° C (Zersetzung).

BEISPIEL 4

Zu einem Bad mit folgender Zusammensetzung
270 g/Liter Nickelsulfat ($NiSO_4$ . $7H_2O$)
40 g/Liter Nickelchlorid ($NiCl_2$ . $6H_2O$)
40 g/Liter Borsäure
0,1 g/Liter 2-Äthylhexylsulfat, Na-Salz, als Netzmittel
wurden 0,15 g/Liter der erfindungsgemäßen Verbindung α-Hydroxipropinsulfonsäure, Natriumsalz zugesetzt.

Diese Zugabe bewirkte eine glänzende Abscheidung in einem Stromdichtebereich von 0,1 - 3 $A/dm^2$ mit bemerkenswerter Glanztiefenstreuung.

BEISPIEL 5

Zu einem Bad mit der Zusammensetzung
250 g/Liter Nickelsulfat ($NiSO_4$ . $7H_2O$)
80 g/Liter Nieckelchlorid ($NiCl_2$ . $6H_2O$)
40 g/Liter Borsäure

0,1 g/Liter 2-Äthylhexylsulfat, Na-Salz, als Netzmittel

wurden 0,15 g/Liter der erfindungsgemäßen Verbindung α-Hydroxipropinsulfonsäure, Natriumsalz, und 1 g/Liter Saccharin (Benzoesäuresulfimid, Na-Salz) zugesetzt.

Man erzielte eine glänzende, merklich eingeebnete Abscheidung in einem Stromdichtebereich von 0,1 - 8 A/dm².

BEISPIEL 6

Zu einem Bad mit folgender Zusammensetzung
270 g/Liter Nickelsulfat (NiSO₄ . 7H₂O)
40 g/Liter Nickelchlorid (NiCl₂ . 6H₂O)
40 g/Liter Borsäure
wurden 1 g/Liter Saccharin, 0,15 g/Liter Pyridiniumpropylsulfonsäurebetain und 0,5 g/Liter Lauryläthersulfat als Netzmittel zugesetzt.

Der Niederschlag war von 2 - 12 A/dm² glänzend, aber nur von 4 - 10 A/dm² eingeebnet und hochglänzend. Das Bad war somit zum Beispiel für Trommelabscheidungen völlig ungeeignet. Durch Zugabe von 0,15 g/Liter der erfindungsgemäßen Verbindung wurde der Bereich 0,5 bis 12 A/dm² gut eingeebnet. Die Einebnung war α-Hydroxipropinsulfonsäure, Natriumsalz in dem genannten Bereich gleichmäßig unabhängig von der Schichtdicke, so daß ein hervorragender dekorativer Effekt zum Beispiel auf Armaturteilen entstand.

BEISPIEL 7

Zu einem Bad mit folgender Zusammensetzung
250 g/Liter Nickelsulfat (NiSO₄ . 7H₂O)
60 g/Liter Natriumchlorid (NaCl)
30 g/Liter Borsäure (H₃BO₃)
0,1 g/Liter 2-Äthylhexylsulfat, Natriumsalz, als Netzmittel
wurden 1 g/Liter Saccharin und 0,3 g/Liter Allylsulfonsäure, Natriumsalz, gegeben. Man erzielte ein gleichmäßig glänzendes Prüfblech ohne Einebnung. Nach Zusatz von 0,2 g/Liter der erfindungsgemäßen Substanz α-Hydroxipropinsulfonsäure, NatriumSalz, erzielte man bis 9 A/dm² eine gleichmäßig glänzende, gut eingeebnete Abscheidung, die wegen ihrer Tiefenstreuung besonders für die Massengalvanisierung in der Trommel geeignet ist. Das Bad bis zu 8 Stunden stabil und zeigte eine unverändert gute Arbeitsweise.

**Patentansprüche**

1. α-Hydroxipropinsulfonsäure und ihre Salze.

2. α-Hydroxipropinsulfonsäure, Natriumsalz.

3. α-Hydroxipropinsulfonsäure, Kaliumsalz.

4. α-Hydroxipropinsulfonsäure, Ammoniumsalz.

5. Saure Nickelbäder, gekennzeichnet durch einen Gehalt an mindestens einer Verbindung gemäß den Ansprüchen 1 bis 4.

6. Saure Nickelbäder gemäß Anspruch 5, enthaltend die Verbindungen in einer Konzentration von 0,005 bis 5 g/Liter, vorzugsweise von 0,05 bis 0,2 g/Liter.

7. Saure Nickelbäder gemäß Ansprüchen 5 und 6, gekennzeichnet durch einen zusätzlichen Gehalt an Grundglänzern in Konzentrationen von 0,1 bis 10 g/Liter.

8. Saure Nickelbäder gemäß Ansprüchen 5 und 6, gekennzeichnet durch einen zusätzlichen Gehalt an Einebnern, vorzugsweise Pyridiniumverbindungen, in Konzentrationen von 0,02 bis 0,25 g/Liter.

9. Saure Nickelbäder gemäß Ansprüchen 5 und 6, gekennzeichnet durch einen zusätzlichen Gehalt an Netzmitteln in Konzentrationen bis zu 10 g/Liter.

10. Verwendung der Verbindungen gemäß Ansprüchen 1 bis 4 in Hochleistungsbädern zur Abscheidung eingeebneter hochglänzender Nickelüberzüge.

11. Verfahren zur Herstellung der Verbindungen gemäß Ansprüchen 1 bis 4, dadurch gekennzeichnet, daß man Propagylaldehyd in wässriger Lösung mit Alkali- oder Ammoniumhydrogensulfit in äquivalenten Mengen bei Raumtemperatur zur Reaktion bringt und die Reaktionsprodukte in an sich bekannter Weise isoliert.

**Claims**

1. α-hydroxypropine sulphonic acid and its salts.

2. α-hydroxypropine sulphonic acid, sodium salt.

3. α-hydroxypropine sulphonic acid, potassium salt.

4. α-hydroxypropine sulphonic acid, ammonium salt.

5. Acid nickel baths, characterized in that they contain at least one compound in accordance with claims 1 to 4.

6. Acid nickel baths in accordance with Claim 5, containing the compounds in concentrations of 0.005 to 5 g/liter, preferably from 0.05 to 0.2 g/liter.

7. Acid nickel baths in accordance with Claims 5 and 6, characterized by an additional content of basic brighteners in concentrations of 0.1 to 10 g/liter.

8. Acid nickel baths in accordance with Claims 5 and 6, characterized by an additional content of leveling agents, preferably pyridinium compounds, in concentrations of 0.02 to 0.25 g/liter.

9. Acid nickel baths in accordance with Claims 5 and 6, characterized by an additional content of wetting agents in concentrations up to 10 g/liter.

10. Use of the compounds in accordance with Claims 1 to 4 in heavy-duty baths for the deposition of bright nickel coatings with good levelling properties.

11. Method for the manufacture of compounds in accordance with Claims 1 to 4, characterized in that propagylaldehyde in an aqueous solution is made to react with alkali or ammonium hydrogen sulphite in equivalent quantities at room temperature, the reaction products being isolated in the way known in the art.

**Revendications**

1. Acide α-hydroxypropyne sulfonique et ses sels.

2. Acide α-hydroxypropyne sulfonique, sel de sodium.

3. Acide α-hydroxypropyne sulfonique, sel de potassium.

4. Acide α-hydroxypropyne sulfonique, sel d'ammoniaque.

5. Bains à nickel acides, caractérisés par une teneur en au-moins un composé selon les revendications 1 à 4.

6. Bains à nickel acides selon la revendication 5, comprenant lesdits composés dans une concentration de 0,005 à 5 g/litre, et de préférence de 0,05 à 0,2 g/litre.

7. Bains à nickel acides selon les revendications 5 et 6, caractérisés par une teneur supplémentaire en produit brillanteur de base dans une concentration de 0,1 à 10 g/litre.

8. Bains à nickel acides selon les revendications 5 et 6, caractérisés par une teneur supplémentaire en agents de lissage, de préférence des composés de pyridinium, dans des concentrations de 0,02 à 0,25 g/litre.

9. Bains à nickel acides selon les revendications 5 et 6, caratérisés par une teneur supplémentaire en agents mouillants dans des concentrations pouvant aller jusqu'à 10 g/litre.

10. Utilisation des composés selon les revendications 1 à 4 dans des bains à haut rendement pour la déposition de couches de nickel brillantes aplanies.

11. Procédé de fabrication des composés selon les revendications 1 à 4, caractérisé par le fait que l'on fait réagir du propagylaldéhyde dans une solution aqueuse avec du sulfite d'hydrogène alcalin ou d'ammoniaque dans des quantités équivalentes à température ambiante et que l'on isole de manière connue en soi les produits de la réaction.